# EUROPEAN PATENT SPECIFICATION

(11) **EP 2 625 284 B1**
(45) Date of publication and mention of the grant of the patent: **28.01.2015**
(21) Application number: 11763703.3
(22) Date of filing: 30.09.2011
(51) Int. Cl.: C12Q 1/68

(54) **METHOD FOR CELL LYSIS IN A RT-PCR REACTION BUFFER**
VERFAHREN FÜR ZELLLYSE IN EINEM RT-PCR-REAKTIONSPUFFER
MÉTHODE DE LYSE CELLULAIRE DANS UN TAMPON DE RÉACTION DE RT-PCR

(30) Priority: 04.10.2010 EP 10186416; 04.10.2010 EP 10186417
(43) Date of publication of application: 14.08.2013
(73) Proprietor: Roche Diagnostics GmbH, 68305 Mannheim (DE); F.Hoffmann-La Roche AG, 4070 Basel (CH)
(72) Inventor: HOFFMANN, Ingrid, 83670 Bad Heilbrunn (DE); WALCH, Heiko, 81667 Muenchen (DE)
(74) Representative: Merkel, Patrick
(86) International application number: PCT/EP2011/067067
(87) International publication number: WO 2012/045668

(56) References cited:
- WO-A1-2009/021215
- YONGZHONG LI ET AL: "An improved one-tube RT-PCR protocol for analyzing single-cell gene expression in individual mammalian cells", ANALYTICAL AND BIOANALYTICAL CHEMISTRY, SPRINGER, BERLIN, DE, vol. 397, no. 5, 20 May 2010 (2010-05-20), pages 1853-1859, XP019839291, ISSN: 1618-2650
- CHRISTOPHER MARLOWE A CAIPANG ET AL: "Rapid diagnosis of vibriosis and white spot syndrome (WSS) in the culture of shrimp, Penaeus monodon in Philippines", VETERINARY RESEARCH COMMUNICATIONS ; AN INTERNATIONAL JOURNAL PUBLISHING TOPICAL REVIEWS AND RESEARCH ARTICLES ON ALL ASPECTS OF THE VETERINARY SCIENCES, KLUWER ACADEMIC PUBLISHERS, DO, vol. 34, no. 7, 28 July 2010 (2010-07-28), pages 597-605, XP019832155, ISSN: 1573-7446
- STAHLBERG A ET AL: "Single-cell gene expression profiling using reverse transcription quantitative real-time PCR", METHODS : A COMPANION TO METHODS IN ENZYMOLOGY, ACADEMIC PRESS INC., NEW YORK, NY, US, vol. 50, no. 4, 1 April 2010 (2010-04-01), pages 282-288, XP026941721, ISSN: 1046-2023, DOI: DOI:10.1016/J.YMETH.2010.01.002 [retrieved on 2010-03-06]
- PEIXOTO ANTÓNIO ET AL: "Quantification of multiple gene expression in individual cells", GENOME RESEARCH, COLD SPRING HARBOR LABORATORY PRESS, WOODBURY, NY, US, vol. 14, no. 10A, 1 October 2004 (2004-10-01), pages 1938-1947, XP002545890, ISSN: 1088-9051, DOI: DOI:10.1101/GR.2890204
- BENGTSSON M ET AL: "Gene expression profiling in single cells from the pancreatic islets of Langerhans reveals lognormal distribution of mRNA levels", GENOME RESEARCH, COLD SPRING HARBOR LABORATORY PRESS, WOODBURY, NY, US, vol. 15, no. 10, 1 October 2005 (2005-10-01), pages 1388-1392, XP002438658, ISSN: 1088-9051, DOI: DOI:10.1101/GR.3820805
- BLIND ET AL: "Differential recruitment of glucocorticoid receptor phospho-isoforms to glucocorticoid-induced genes", JOURNAL OF STEROID BIOCHEMISTRY AND MOLECULAR BIOLOGY, ELSEVIER SCIENCE LTD., OXFORD, GB, vol. 109, no. 1-2, 19 January 2008 (2008-01-19), pages 150-157, XP022534002, ISSN: 0960-0760, DOI: 10.1016/J.JSBMB.2008.01.002
- SASTRE J ET AL: "Age-associated oxidative damage leads to absence of gamma-cystathionase in over 50% of rat lenses: Relevance in cataractogenesis", FREE RADICAL BIOLOGY AND MEDICINE, ELSEVIER SCIENCE, US, vol. 38, no. 5, 1 March 2005 (2005-03-01), pages 575-582, XP025352055, ISSN: 0891-5849 [retrieved on 2005-03-01]
- AGÜERO MONTSERRAT ET AL: "A highly sensitive and specific gel-based multiplex RT-PCR assay for the simultaneous and differential diagnosis of African swine fever and Classical swine fever in clinical samples.", VETERINARY RESEARCH 2004 SEP-OCT LNKD- PUBMED:15369658, vol. 35, no. 5, September 2004 (2004-09), pages 551-563, XP002663152, ISSN: 0928-4249
- GRIMMLER C ET AL: "Transcriptional analysis of catabolite repression in Clostridium acetobutylicum growing on mixtures of d-glucose and d-xylose", JOURNAL OF BIOTECHNOLOGY, ELSEVIER SCIENCE PUBLISHERS, AMSTERDAM, NL, vol. 150, no. 3, 29 September 2010 (2010-09-29), pages 315-323, XP027483999, ISSN: 0168-1656, DOI: 10.1016/J.JBIOTEC.2010.09.938 [retrieved on 2010-09-29]
- CHEUNG T K W ET AL: "Evaluation of novel H1N1-specific primer-probe sets using commercial RT-PCR mixtures and a premixed reaction stored in a lyophilized format", JOURNAL OF VIROLOGICAL METHODS, ELSEVIER BV, NL, vol. 165, no. 2, 1 May 2010 (2010-05-01), pages 302-304, XP026993889, ISSN: 0166-0934 [retrieved on 2010-02-06]
- TETSUTARO HAYASHI ET AL: "Single-cell gene profiling of planarian stem cells using fluorescent activated cell sorting and its "index sorting" function for stem cell research", DEVELOPMENT, GROWTH & DIFFERENTIATION, vol. 52, no. 1, 1 January 2010 (2010-01-01), pages 131-144, XP55012184, ISSN: 0012-1592, DOI: 10.1111/j.1440-169X.2009.01157.x
- WARREN LUIGI ET AL: "Transcription factor profiling in individual hematopoietic progenitors by digital RT-PCR", PROCEEDINGS OF THE NATIONAL ACADEMY OF SCIENCES OF USA, NATIONAL ACADEMY OF SCIENCE, WASHINGTON, DC; US, vol. 103, no. 47, 1 November 2006 (2006-11-01), pages 17807-17812, XP002479982, ISSN: 0027-8424, DOI: 10.1073/PNAS.0608512103
- STÅHLBERG ANDERS ET AL: "Quantitative transcription factor analysis of undifferentiated single human embryonic stem cells.", CLINICAL CHEMISTRY DEC 2009 LNKD- PUBMED:19815608, vol. 55, no. 12, December 2009 (2009-12), pages 2162-2170, XP55012180, ISSN: 1530-8561
- YUAN GONG ET AL: "Massively parallel detection of gene expression in single cells using subnanolitre wells", LAB ON A CHIP, vol. 10, no. 18, 1 January 2010 (2010-01-01), page 2334, XP55012182, ISSN: 1473-0197, DOI: 10.1039/c004847j
- SELVARAJU S B ET AL: "Evaluation of three analyte-specific reagents for detection and typing of herpes simplex virus in cerebrospinal fluid", DIAGNOSTIC MICROBIOLOGY AND INFECTIOUS DISEASES, ELSEVIER SCIENCE PUBLISHING CO., AMSTERDAM, NL, vol. 63, no. 3, 1 March 2009 (2009-03-01), pages 286-291, XP025940314, ISSN: 0732-8893, DOI: 10.1016/J.DIAGMICROBIO.2008.11.013 [retrieved on 2009-02-11]

### SEQUENCE LISTING

<110> Roche Diagnostics GmbH F. Hoffmann-La Roche AG
<120> Method for cell lysis in a RT-PCR reaction buffer
<130> 30629 WO
<150> EP 10186417.1
   <151> 2010-10-04
<150> EP 10186416.3
   <151> 2010-10-04
<160> 12
<170> PatentIn version 3.5
<210> 1
   <211> 20
   <212> DNA
   <213> Artificial Sequence
<220>
   <223> forward primer
<400> 1
   agcttgtcat caacgggaag 20
<210> 2
   <211> 21
   <212> DNA
   <213> Artificial Sequence
<220>
   <223> reverse primer
<400> 2
   tttgatgtta gtggggtctc g 21
<210> 3
   <211> 20
   <212> DNA
   <213> Artificial Sequence
<220>
   <223> forward primer
<400> 3
   catgaggtcg ggtggaagta 20
<210> 4
   <211> 20
   <212> DNA
   <213> Artificial Sequence
<220>
   <223> reverse primer
<400> 4
   gcctgtttcc gtaacctcaa 20
<210> 5
   <211> 20
   <212> DNA
   <213> Artificial Sequence
<220>
   <223> forward primer
<400> 5
   ctgtcttgcc ttcgtgctct 20
<210> 6
   <211> 20
   <212> DNA
   <213> Artificial Sequence
<220>
   <223> reverse primer
<400> 6
   agcagggcta tcaaccaaaa 20
<210> 7
   <211> 20
   <212> DNA
   <213> Artificial Sequence
<220>
   <223> forward primer
<400> 7
   aaggccaacc gtgaaaagat 20
<210> 8
   <211> 21
   <212> DNA
   <213> Artificial Sequence
<220>
   <223> reverse primer
<400> 8
   gtggtacgac cagaggcata c 21
<210> 9
   <211> 20
   <212> DNA
   <213> Artificial Sequence
<220>
   <223> forward primer
<400> 9
   tacgcctgca gagttaagca 20
<210> 10
   <211> 23
   <212> DNA
   <213> Artificial Sequence
<220>
   <223> reverse primer
<400> 10
   ggttcaaatg aatcttcaga gca 23
<210> 11
   <211> 21
   <212> DNA
   <213> Artificial Sequence
<220>
   <223> forward primer
<400> 11
   gccgctagag gtgaaattct t 21
<210> 12
   <211> 19
   <212> DNA
   <213> Artificial Sequence
<220>
   <223> reverse primer
<400> 12
   cgtcttcgaa cctccgact 19

## Description

### Field of the Invention

The invention relates to the field of RNA expression analysis by means of PCR. More specifically, the present invention provides a method in order to perform expression analysis starting from a material of only a few cells, and subsequent direct analysis of said sample RNA by means of real time PCR.

### Background of the Invention

In the past decades, PCR has become the "working horse" for the analysis of DNA since it enables exponential amplification of nucleic acids. In particular real time PCR (also termed qPCR) has become a powerful tool since it enables simultanous analysis of the amplified nucleic acid during the amplification, or without intermediate opening, directly subsequent to the amplification reaction by means of melting curve analysis.

Moreover, automatization of PCR as well as RT-PCR has made significant progress, since qPCR systems are now available which enable performance of 96, 384 or 1536 reactions in parallel in microtiter plate formates. Systems have also become more user friendly. For example, microtiter plates are commercially available, wherein each reaction vessel already comprises the compounds necessary to perform PCR or RT-PCR amplification or amplification and detection in a freeze dried form, and the customer only has to add the sample comprising the nucleic acid to be analyzed, prior to the actual reaction itself. An alternative system is provided by Advalytics (AG 480F) which enables PCR amplification and detection on glass slides.

Nevertheless, it is still a challenge to further improve the work flow for PCR and RT-PCR analysis, in particular, if analysis can only be performed on RNA originating from only a small number of cells. In traditional real time PCR, DNA or RNA is first isolated from cells in a time consuming procedure that can lead to a loss of material. After harvesting, the cells are lysed and the DNA is at least partially purified from the lysate, because otherwise, PCR amplification might be inhibited at least quantitatively.

Stahlberg A. et al. "Single-cell gene expression profiling using reverse transcription quantitative real-time PCR", April 2010, METHODS 50 (4): 282-288) reports a method to collect dispersed single cells either by glass capillaries or flow cytometry, followed by quantitative mRNA profiling using reverse transcription and real-time PCR.

In this context, the technical problem underlying the present invention was to provide an improved and automatable high throughput method which allows for a further simplified nucleic acid analysis protocol.

### Summary

The present invention provides a method for amplification of an RNA target nucleic acid, comprising the steps of
a) transfering a liquid sample with a first volume of less than 2 µl comprising one or more living eukaryotic cells into a vessel,
b) adding to said vessel a one-step RT-PCR reaction buffer comprising a thermostable DNA polymerase capable of performing a one-step RT-PCR, and dNTPs, with a second volume, whereas said second volume is at least 2x as large as said first volume,
b') incubating said sample at a temperature between 37°C and 65°C for 30 seconds to 5 min, c) heating said vessel for at least 20 seconds at at least 90°C,
d) amplifying said target in said vessel by means of a polymerase chain reaction with a thermostable DNA polymerase capable of performing a one-step RT-PCR without performance of an intermediate purification step,
wherein the ratio of the number of said living cells of step a) versus the liquid volume in which the polymerase chain reaction of step d) is not greater than 2 cells/µl, said ratio is at least 1 cell/25µl.

Said one-step-RT-PCR reaction buffer of step b) may in addition comprise at least one pair of amplification primers and optionally at least one labeled hybridization probe or a double strand DNA binding fluorescent compound.

Alternatively, said vessel comprises a dry composition of at least one pair of PCR amplification primers and optionally either at least one labeled hybridization probe or a double strand DNA binding fluorescent compound.

In one embodiment, the activity of said thermostable DNA Polymerase is thermally activated during step c).

Also in one embodiment which is not mutually exclusive with the embodiment disclosed above, said polymerase is Tth Polymerase.

In one embodiment of the inventive method, said liquid comprising at least one or more living cells has been gained by a cell sorting method prior to step a).

Preferably, the nucleic acid that shall be amplified is RNA. It is also within the scope of the present invention, if the target RNA is only expressed in very low amounts and/or transcribed from single copy DNA

In another aspect, a kit comprising a plurality of reaction vessels designed to fit into a thermocycler instrument, and a PCR reaction buffer comprising a thermostable DNA polymerase capable of performing a one-step RT-PCR and dNTPs is provided.

In one aspect, said kit further comprises at least one pair of amplification primers, and optionally either at least one labeled hybridization probe or a double strand DNA binding fluorescent compound.

In an alternative aspect, said reaction vessels within said kit comprise a dry composition of at least one pair of PCR amplification primers and optionally either at least one labeled hybridization probe or a double strand DNA binding fluorescent compound

Said plurality of reaction vessels may be physically connected with each other in a form of a microtiter plate or a linear strip of reaction vessels.

In addition said thermostable polymerase within such a kit may preferably be thermally activiated by means of incubtion for at least 1 minute at 90°C.

### Detailed Description

Generally speaking, the present invention provides a method which enables lysis of a cell sample in a liquid environment that later on is used directly for RNA expression analysis by means of applying a one-step-RT-PCR without any intermediate purification step or complex liquid handling procedures. More precisely, the present invention provides a method for amplification of an RNA target nucleic acid, comprising the steps of
a) transfering a liquid sample with a first volume of less than 2 µl comprising one or more living eukaryotic cells into a vessel,
b) adding to said vessel a one-step RT-PCR reaction buffer comprising a thermostable DNA polymerase capable of performing a one-step RT-PCR, and dNTPs, with a second volume, whereas said second volume is at least 2x as large as said first volume,
b') incubating said sample at a temperature between 37°C and 65°C for 30 seconds to 5 min, c) heating said vessel for at least 20 seconds at at least 90°C,
d) amplifying said target in said vessel by means of a polymerase chain reaction with a thermostable DNA polymerase capable of performing a one-step RT-PCR without performance of an intermediate purification step,
wherein the ratio of the number of said living cells of step a) versus the liquid volume in which the polymerase chain reaction of step d) is not greater than 2 cells/µl, said ratio is at least 1 cell/25µl.

According to one aspect of the present invention it is possible that all steps a), b), c) and d) are performed within the same reaction vessel.

As it will be demonstrated by the examples, the present disclosure provides a method which is surprisingly applicable to perform a 1-step-RT-PCR analysis starting from only one single living cell as original sample material. Moreover it is possible to reproducibly amplify and analyze target DNA from a single copy gene from only a few cells or even one single cell.

The target nucleic acid may be any RNA, including but not limited to mRNA for the purpose of monitoring respective RNA expression levels. Thus it is required to amplify the RNA target nucleic acid with a thermostable polymerase that comprises both RNA template dependent polymerase activitiy (Reverse Transcriptase activity) and DNA template dependent activity. A prominent example for such an enzyme is Tth DNA polymerase (Roche Applied Science Cat. No: 11 480 022 001). Alternatively the thermostable DNA polymerase comprising reverse transcriptase activity as well as DNA dependent polymerase activity may be a mixture of an enzyme with reverse transcriptase activity and a DNA dependent DNA polymerase activity. For example, the C. therm Polymerase system (Roche Applied Science Cat. No: 12 016 346 001) consists of a mixture of C.therm polymerase and Taq Polymerase.

The elevated temperatures of Tth DNA Polymerase (optimum +55°C to +70°C, maximum +95°C) activity overcomes the problems posed by RNA secondary structure. Resulting cDNA can be amplified by PCR using the same enzyme in the presence of Mg²⁺-ions. The ability of Tth DNA Polymerase to perform both reverse transcription and DNA amplification at elevated temperatures allows this enzyme to be used for quantitative RT-PCR, cloning, and gene expression analysis. Tth DNA Polymerase is used for RT-PCR amplification of RNA up to 1 kb.

Tth DNA Polymerase activity is resistant to prolonged incubations at high temperatures (+95°C), and can therefore be used for PCR amplification.

In the presence of Manganese-ions (Mn²⁺) Tth DNA Polymerase has a very efficient intrinsic reverse transcriptase (RT) activity, which is much higher than the activity reported for E. coli DNA polymerase and Taq DNA polymerase (Saiki, R.K., et al., Science 239 (1988) 487-491).

### Step a)

The one or more living cells according to step a) are eukaryotic cells of human, animal or plant origin. The cells may be derived from cell lines, blood or biopsies.

The liquid sample containing the cells may be any liquid, buffer or medium in which said cells survive for at least a certain period of time, which is preferably longer than 30 minutes. Such a liquid, for example may be a medium in which cells living and growing in suspension have been successfully cultivated. In case of adherent cells, the liquid may be a buffer, in which the cells have been detached from the solid support. Preferably, however such a buffer is free of any proteases which might have an inhibitory effect on the subsequent polymerase mediated PCR or RT-PCR amplification reaction. Such an effect, if observed, can be avoided by subjecting the cells to an additional washing step prior to deposition into the vessel.

The transfer of the liquid into the vessel may be achieved either manually by means of first preparing an appropriate dilution series of a cell sample and then pipeting an equivalent of only one or several cells into said vessel. Preferably, the transfer is achieved using an appropriate automated pipetting station or, mostly preferred, a cell sorter. Such cell sorting machines are well known in the art and commercially available from a number of different manufacturers. Due to the underlying technology of cell sorters, it will happen from time to time that large particles of cell debris are mistakenly recognized by the cell sorters as cells. Due to this effect, in case of single cell analysis some samples may give no results during the subsequent PCR or RT-PCR reaction.

Preferably, the number of cells transferred to the reaction vessel should be limited since without any purification step, the presence of cellular debris in higher concentrations may inhibit the subsequent amplifcation reaction. Advantageously, the number of cells transferred to the reaction vessel is adjusted so that step d) as disclosed below is performed in such a way that the sample does not exceed a ratio of 2 cell equivalents/µl.

Also preferably, the number of cells transferred to the reaction vessel should not be to low, because otherwise it may become difficult to perfom a PCR or RT-PCR reaction for the amplification of RNAs which are only expressed with low abundancy. Thus, the number of cells transferred to the reaction vessel is adjusted so that step d) as disclosed below is performed in such a way that the sample comprises a ratio of at least 1 cell equivalents/ 25 µl.

The volume of the liquid is sufficiently small such that addition of the 1-step-RT-PCR reaction buffer in step b) and - if required - addition of further compounds required for the subsequent amplification reaction finally result in a final volume which is still reasonable small for performance of said RT-PCR.

In case of automated deposition of the liquid containing the one or more cells can be very low in sub-µl range. In particular, if a cell sorter is used, a volume containing only one cell equivalent is about less than 100 nl. Even if in the latter case the sample is dried out, it has been proven by the inventor that the new method is still effective. In the particular embodiment of single cell analysis, the final volume preferably does not exceed 25µl.

The reaction vessel may be any type of reaction vessel, in which an amplification reaction can be performed. Therefore, the only essential limitation is that the vessel has sufficient heat resistance, since during the thermocycling protocol it will become repeatedly exposed to high temperatures of 90°C or above.

In one embodiment, the reaction vessel is a well of a microtiter plate which is suited or designed to be placed into a thermocycler instrument. This allows to execute the method of the present invention on a number of samples in a highly parallel manner. Microtiter plates comprising 96, 384, and 1536 wells are known in the art and commercially available from a multitude of different suppliers. Microtiter plates available in the art allow for reaction volumes of at least 2 µl. Such microtiter plates can be subjected to high temperature of at least 90°C by means of placing them on an appropriate heating block, or, alternatively, directly into a PCR Thermocycler instrument which is designed to incorporate microtiter plates.

In a second embodiment the reaction vessel may be a single reaction tube or a reaction tube which is a part of a strip of reaction tubes that are connected to each other so that they can jointly be placed into the heating block of a thermocycler instrument. In a further embodiment, the reaction vessel is a specific capillary which can be placed into a capillary LightCycler instrument (Roche Applied Science Cat. No. 023 531 414 001).

### Step b)

In the context of the present invention, the term "1-step-RT-PCR reaction buffer" which is added at step b) of the present invention is understood as any liquid in which the sample later on can be subjected to an RT-PCR reaction without any intermediate purification step. The said second volume of the reaction buffer added should be at least twice (2x) as large and preferably 5 times (5x) as large as the first volume. This will allow for efficient amplification of the target nucleic acid during the subsequent 1-step RT-PCR reaction.

The "1-step RT-PCR reaction buffer" comprises at least a thermostable DNA polymerase comprising both reverse transcriptase and DNA dependent DNA polymerase activity, and a mixture of desoxynucleoside-triphosphates (dNTPs). In one embodiment, the "1-step-RT-PCR reaction buffer" already contains compounds which are further required for the performance of a PCR reaction. Thus said "1-step RT-reaction buffer" may further comprise at least one appropriate pair of amplification primers. The "1step RT-PCR reaction buffer may also comprise an appropriate pH buffering compound (e.g. Tris), a Mg2+ salt, a Mn2+ salt, a hot start component and the like.

In a specific embodiment, the "1-step-RT-PCR reaction buffer" may already comprise compounds that are required for monitoring the amplification of the target DNA in real time. In particular, these compounds are either fluorescent hybridization probes or a double stranded DNA binding dye. The specifics of various possible detection formats will be discussed below.

Alternatively, it is also within the scope of the invention, if some, any or all of the aforementioned components or any other additional compounds are added subsequently to the lysis step c) but prior to the performance of the actual amplification reaction according to step d).

### Step c)

According to the present invention, lysis of the cells takes place within the 1-step-RT-PCR reaction buffer, without any prior addition of a specific lysis step reagent conventionally used in the art. Rather, lysis of the one or more living cells takes place by means of incubating the sample for a period of at least 20 seconds at at least 90°C. Usually, a period of less than 1 min of high temperature incubation is sufficient for a complete lysis of a moderate number of cells (not more than 64 cells) which enables for subsequent amplification and detection of transcripts expressed with low abundancy. However, it is also within the scope of the present invention, if said period is prolonged up to a period of 30 but preferably not more than 15 minutes.

The temperature used for cell lysis should not exceed 100°C and preferably be 95°C or less because at higher temperatures there is an increasing risk that the components contained in the reaction buffer which are required for the subsequent PCR reaction are destroyed. For example even thermostable DNA polymerases such as Taq DNA polymerase are becoming substantially denatured or degraded at temperatures above 100°C.

### Step d)

As evidenced by the examples, a 1-step-RT-PCR reaction can be performed using the lysate directly without intermediate purification step. Dependent on the embodiment, however it is within the scope of the present invention, if additional compounds required for said reaction are added to the sample subsequent to the lysis.

Since the present invention is applicable for analysis of nucleic acids originating from only very few or even single cells, it is advantageous for the design of an experiment according to the present invention, if consideration is given to the ratio between the number of cells analyzed and the volume in which the actual RT-PCR reaction according to step d) takes place. On the one hand, the reaction should be performed in a minimal volume in order to achieve an optimal degree of sensitivity if such a low amount of starting material shall be analyzed. Thus, it has been proven to be advantageous, if the ratio of the number of said cells of step a) versus the liquid volume in which the polymerase chain reaction of step d) is performed is at least 1 cell/ 20µl reaction volume.

Since there is no intermediate purification step, the lysed sample will contain cellular debris which may interfere with the efficiency of the PCR reaction. In this context, it has been proven to be advantageous, if the ratio of the number of said living cells or cell equivalents of step a) versus the liquid volume in which the polymerase chain reaction of step d) is performed is not greater than 2 cells/µl. Even more advantageous is a ratio between 1 cell/25µl and 2 cells/µl. As it has been determined experimentally a ratio within this range enables single copy analysis on DNA originating from only 1 single cell as well as a much higher cell numbers.

The compounds used for 1-step RT-PCR reaction may be conventional compounds used in the art: The reactive reaction set up comprises the target RNA, dNTPs, a thermostable DNA Polymerase which is also capable of performing 1-step RT PCR in order to use the inventive method for monitoring of gene expression, and at least one pair of amplification primers.

Advantageously, the primers are designed in such a way that the generated amplification product is relatively small. Amplification products with a size of up to 1 kb have been quantitatively generated with the method according to the present invention, but an amplicon sizes of less than 300 bp or even less than 120 bp are highly preferred.

Also advantageously, the primers are designed in such a way that they span an intron sequence, i.e. one part of a respective primer is directed against a first exon and the other part of said primer is directed against a second consecutive exon. Such a design enables to create specifically amplification product derived from the RNA in the sample and excludes the possibility to generate amplification products that may be derived from the respective genomic DNA fragment.

The reverse transcription part of the 1-step-RT PCR does not require a separate programming within a thermocycler instrument. Instead, the reverse transcription step takes place during the initial ramping, annealing and elongation steps of the thermocycling protocol. This is in accordance with observations disclosed in EP 1 978 109.

However, for small sample volumes of 2µl or less, the inventors have surprisingly observed, that the detection of expression is even more sensitive, in case of a preincubation step prior to the actual thermal lysis step at a temperature which is around the optimum for a reverse transcriptase reaction. Thus, said sample has a volume of less than 2 µl and between step b) and c) said sample is incubated at a temperature between 37°C and 65°C for 30 seconds to 5 min. Probably such a positive effect is due to the small sample volume of less than 2 µl, which results in an immediate drying and subsequent destruction of the membranes of the few sorted cells. As a consequence, the cellular RNA may become available for the reverse transcription reaction.

Analysis of the amplified DNA may subsequently be achieved usually by means of Gel Electrophoresis. However, in a more important embodiment, the RT-PCR reaction may be a real time PCR reaction, wherein the progress of amplification is continously monitored using, for example any of the following detection formates:

### - TaqMan Hydrolysis probe format:

A single-stranded Hybridization Probe is labeled with two components. When the first component is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3' exonuclease activity of the Taq Polymerase during the subsequent elongation phase. As a result the excited fluorescent component and the quencher are spatially separated from one another and thus a fluorescence emission of the first component can be measured. TaqMan probe assays are disclosed in detail in US 5,210,015, US 5,538,848, and US 5,487,972. TaqMan hybridization probes and compound mixtures are disclosed in US 5,804,375.

In a specific embodiment, the Taqman hybridization probes are UPL probes from the Universal Probe Library as available from Roche Applied Sciences (Cat. 2010/2011, p. 577).

### - Molecular Beacons:

These hybridization probes are also labeled with a first component and with a quencher, the labels preferably being located at both ends of the probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).

### - FRET hybridization probes:

The FRET Hybridization Probe test format is especially useful for all kinds of homogenous hybridization assays (Matthews, J.A., and Kricka, L.J., Analytical Biochemistry 169 (1988) 1-25). It is characterized by two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected. Alternatively to monitoring the increase in fluorescence of the FRET acceptor component, it is also possible to monitor fluorescence decrease of the FRET donor component as a quantitative measurement of hybridization event.

In particular, the FRET Hybridization Probe format may be used in real time PCR, in order to detect the amplified target DNA. Among all detection formats known in the art of real time PCR, the FRET-Hybridization Probe format has been proven to be highly sensitive, exact and reliable (WO 97/46707; WO 97/46712; WO 97/46714). As an alternative to the usage of two FRET hybridization probes, it is also possible to use a fluorescent-labeled primer and only one labeled oligonucleotide probe (Bernard, P.S., et al., Analytical Biochemistry 255 (1998) 101-107). In this regard, it may be chosen arbitrarily, whether the primer is labeled with the FRET donor or the FRET acceptor compound.

### - Double Strand DNA binding dye format:

It is also within the scope of the invention, if real time PCR is performed in the presence of an additive according to the invention in case the amplification product is detected using a double stranded nucleic acid binding moiety. For example, the respective amplification product can also be detected according to the invention by a fluorescent DNA binding dye which emits a corresponding fluorescence signal upon interaction with the double-stranded nucleic acid after excitation with light of a suitable wavelength. The dyes SybrGreenI and SybrGold (Molecular Probes) are frequently used in the art. Another particularly useful dye is the LightCycler 480 Resolight dye (Roche Applied Science Cat. No: 04 909 640 001).

### Hot start PCR

The PCR reaction set up may contain some compounds which are providing a hot start effect, i.e. the inhibition of unspecific primer annealing and subsequent elongation at ambient temperature, which occasionally results in unspecific amplification products such as primer dimer formation. Upon temperature increase, this inhibition becomes eliminated due to release of the hot start compound from any binding partner with the consequence that the thermostable DNA Polymerase is becoming thermally activiated and specific polymerase catalyzed primer extension can occur. Many examples for such compounds are known in the art. A specific examples is given in US 5,338,671, which discloses a Polymerase antibody as a hot start compound. More recent examples for such hot start compounds are disclosed in EP 1 989 324 A and EP 2 163 556.

Said hot start compounds may be added to the sample subsequent to lysis together with the polymerase and any other RT-PCR compounds prior to step d). Preferably, however, said hot start compounds are included already within the "1-step-RT-PCR reaction buffer" that is being added during step b). As a consequence the thermal acitivation of the thermostable DNA polymerase can already be achieved through the incubation at at least 90°C during step c).

In a particular embodiment, the DNA polymerase is reversibly inactivated as a result of a chemical modification. More precisely, heat labile blocking groups are introduced into the polymerase which renders the enzyme inactive at room temperature (US 5,773,258). These blocking groups are removed at high temperature during a pre-PCR step such that the enzyme is becoming activated. Such a heat labile modification, for example can be obtained by coupling Citraconic Anhydride or Aconitric Anhydride to the Lysine residues of the enzyme (US 5,677,152).

In a preferred embodiment, the hot start feature is achieved by using Tth DNA Polymerase in combination with Aptamers. Tth DNA Polymerase is a thermostable enzyme with RNA-dependent reverse transcriptase activity and DNA-dependent polymerase activity, allowing the combination of reverse transcription (RT) and PCR in a single-tube reaction.

Aptamers are dedicated oligonucleotides that bind to the active center of the polymerase and prevent attachment to nucleic acid targets at temperatures below the optimal reaction temperature of the Tth enzyme. Therefore, no primer elongation occurs during reaction setup and the subsequent heating phase prior to the RT step. At higher temperatures, the Aptamers are released from the enzyme, and RT or DNA polymerization can be initiated. The recommended incubation temperature for RT with Tth DNA Polymerase (+61°C) can overcome secondary structures of RNA. This results in highly specific and efficient cDNA synthesis, which leads to highly specific and sensitive PCR.

Hot start with Aptamers is highly effective and very convenient because it does not require additional incubation steps, pipetting steps, or an extension of reaction time. The hot start protocol with Aptamers does not interfere with other enzymatic processes, the online detection of amplification products, or subsequent handling steps.

### Microtiter plates comprising a dry composition of PCR reagents

As disclosed above, microtiter plates may be used in order to perform a method according to the present invention. If this is the case, each vessels or reaction well of the microtiter plate may already comprise on its surface a dry composition of reagents which are subsequently required for PCR. The dry composition of the master mix is resolved by means of addition of the "1-step-RT-PCR reaction buffer" in step b). In the context of the present invention the phrase "dry composition" is used to emphasize that the amount of solvent, preferably of aqueous solvents is reduced below 5 weight %.

For example, such a dry composition may comprise or only consist of at least one pair of amplification primers. In this case, the "1-step-RT-PCR reaction buffer" does not include any amplification primers. Each well of a microtiter plate may comprise the same pair of amplification primers thereby enabling parallel analyis of multiple samples, or, substantially each well may comprise different pairs of amplification primers, thereby enabling a multiparametric analysis of one or only a few different samples. Of course, the plate layout can also be designed according to a mixture of the two concepts as well as for duplicate, triplicate or quadruplicate analysis. Methods for producing dry compositions of nucleic acids such as PCR amplification primers are well known in the art and include but are not limited to methods of freeze drying, lyophyllization or vacuum drying. WO 2008/36544 describes the use of so-called filler materials in order to provide dried compositions, said filler materials are e.g. carbohydrates such as FICOLL™, sucrose, glucose, trehalose, melezitose, DEXTRAN™ or mannitol, proteins such BSA, gelatin or collagen and polymers such as PEG or polyvinyl pyrrolidone (PVP). Freeze-drying (US 5,593,824) or vacuum drying (US 5,565,318) have been disclosed for drying the biological materials in a carbohydrate polymer matrix.

In addition, such dry compositions may optionally comprise either at least one labeled hybridization probe or a double strand DNA binding fluorescent compound in order to enable real time PCR monitoring. Furthermore, said dried composition may additionally comprise a thermostable DNA polymerase and/or dNTPs. In this case, the "1-step-RT-PCR reaction buffer" does not include any reagents which are part of the dry composition.

Several methods of producing dry compositions comprising proteins or enzymes are also disclosed in the art. Lyophilisation or freeze-drying is a well established technique towards storage of proteins that is disclosed in many state of the art documents (e.g. Passot, S., et al., Pharmaceutical Development and Technology 12 (2007) 543-553; Carpenter, J.F., et al., Pharmaceutical Research 14 (1997) 969-975; Schwegman, J.J., et al., Pharmaceutical Development and Technology 10 (2005) 151-173). In particular, US 7,407,747 discloses that a Taq polymerase can be dried in a mixture consisting of buffer solution, nucleotides, BSA and trehalose. Also, US 2010/0159529 discloses that the addition of an aptamer to the liquid solution enhanced the stability of the Taq polymerase, wherein said stabilization was good enough not only to dry, but also to store the dried mixture.

As will be understood by the skilled artisan, the disclosed method encompasses a number of variations. For example, if the dry composition comprises only amplification primers, a 1-step-RT-PCR master mix comprising the thermostable DNA Polymerase, dNTPs and all other PCR compounds necessary for amplification may be added as "1-step-RT-PCR reaction buffer" during step b. Optionally either the "1-step-RT-PCR reaction buffer" or the 1-step-RT-PCR master mix may in addition comprise a means for monitoring amplification in real time such as a fluorescently labeled hybridization probe, or, alternatively a fluorescent double strand DNA binding dye.

In another example, the dry composition may comprise all compounds necessary for amplification, i.e. at least one pair of amplification primers, the thermostable DNA Polymerase, dNTPs and optionally at least one flourescently labeled hybridization probe or fluorescent double strand DNA binding dye. Then, the "1-step-RT-PCR reaction buffer" of step b) may be simply water, or, if necessary may comprise additional accessory compounds such as a pH buffering system (e.g. Tris salt), Mg2+salt and the like.

### Kits

The present disclosure also provides a new type of kits for performing real time PCR analysis. The kits comprise a reagent component and a disposable component, which together can be used and are specifically adapted for any of the methods disclosed above.

Thus such a kit comprises a plurality of reaction vessels designed to fit into a thermocycler instrument, and a one-step-RT-PCR reaction buffer comprising a thermostable DNA polymerase comprising reverse transcriptase and DNA dependent DNA polymerase activitiy or a mixture of polymerases which together provide both such activites, and dNTPs. Thus, such a kit for the first time provides to the scientist a useful tool containing a complete set of all reagents and disposables necessary for gene expression analysis.

Prominent examples for suitable enzymes are Tth DNA polymerase (Roche Applied Science Cat. No: 11 480 022 001) or the C. therm Polymerase system (Roche Applied Science Cat. No: 12 016 346 001). In a highly preferred embodiment a hot start feature is implemented by using Tth DNA Polymerase in combination with Aptamers. Tth DNA Polymerase is a thermostable enzyme with RNA-dependent reverse transcriptase activity and DNA-dependent polymerase activity, allowing the combination of reverse transcription (RT) and PCR in a single-tube reaction. Preferably, said thermostable polymerase within such a kit may be thermally activiated by means of incubation for at least 1 minute at 90°C.

Preferably, the reaction vessels are physically connected with each other in a form of a microtiter plate. The microtiter plate may preferably be a 96- 384- or 1536-well mcrotiter plate. Alternatively, said reaction vessels are physically connected to each other in the form of a linear strip of reaction vessels.

In one embodiment, said kit further comprises at leat one pair of amplifciation primers, and optionally either at least one labeled hybridization probe or a double strand DNA binding fluorescent compound. These reagents are stored within separate vessels and may be added to the PCR reaction buffer prior to the start of the experiment.

In an alternative embodiment, said reaction vessels within said kit comprise a dry composition of at least one pair of PCR amplification primers and optionally either at least one labeled hybridization probe or a double strand DNA binding fluorescent compound.

### Examples

### Example 1

### qPCR for amplification of the GAPDH gene and the RPLI13A gene from sorted mouse hybridoma cells

A defined number of Mouse Hybridoma cells was deposited into separate wells of a 96 well microtiter plate using a Cell Sorter (Beckton Dickinson, FACS Aria I) in such a way that the liquid beam was always oriented into the center of the well. Due to the underlying technology of the Cell Sorter, however, it could not be excluded that a minor percentage of the sorted particles were not intact whole cells but cellular debris. Thus, in the following, the number of sorted material will be termed cell equivalent.

Cells sorted as disclosed were distributed into a 96 well microtiter plate (Roche Applied Science Cat. No: 04 729 692 001) designed for the LC480 real time PCR instrument (Roche Applied Science Cat. No: 05 015 278 001) according to the following pipetting scheme:
1 cell equivalent/well of column 1-4
2 cell equivalents/well in column 5-6
4 cell equivalents/well in column 7-8
8 cell equivalents/well in column 9
16 cell equivalents/well in column 10
32 cell equivalents/well in column 11
64 cell equivalents/well in column 12

To each well, a master mix was added which contained
0.4 µM Forward primer agcttgtcatcaacgggaag (SEQ ID NO: 1)
0.4 µM Reverse primer tttgatgttagtggggtctcg (SEQ ID NO: 2)

0.2 µM UPL Probe (RocheApplied Science Cat. No: 04 685 075 001, No. 9)
1x LC480 Probe Master (Roche Applied Science Cat. No: 04 902 343 001)

The forward and reverse primer were designed to amplify the mouse GAPDH gene, which is known to be present in the mouse genome in high copy numbers.

On a separate plate the same master mix was added, but primers and probe were designed to amplify the gene RPLI13A, which is present in only 12 copies of the mouse genome. Primers and probe were as follows:
0.4 µM Forward primer catgaggtcgggtggaagta (SEQ ID NO: 3)
0.4 µM Reverse primer gcctgtttccgtaacctcaa (SEQ ID NO: 4)
0.2 µM UPL Probe (RocheApplied Science Cat. No: 04 686 993 001, No.25)

The LightCycler Probe Master comprises the thermostable FastStart DNA polymerase, which is a hot start enzyme that is chemically modified. Activation is induced by means of removing said modification through incubation at high temperature.

qPCR was performed in an LC480 real time PCR instrument according to the following thermocycling protocol:

| | | | |
|---|---|---|---|
| Preincubation: | 1x | 95°C | 10' |
| Denaturation | 45x | 95°C | 10" |
| Annealing | 45x | 60°C | 30" |
| Elongation | 45x | 72°C | 1" |
| Cooling ramp rates | 2.2°C/s | | |
| Heating ramp rates | 4.4°C/s | | |

Detection of amplification signals and calculation of cp values (low cp values indicating a high level of amplification) was performed according to the instructions of the Manufacturer's manuals.

The following table discloses the average cp values obtained for the different cell numbers analyzed:

| Number of cells | Average cp Value | |
|---|---|---|
| | GAPDH | RPLI13A |
| 1 | 33,00 | 36.10 |
| 2 | 31.40 | 34,00 |
| 4 | 31.28 | 33,92 |
| 8 | 29,38 | 33,35 |
| 16 | 29,02 | 31,67 |
| 32 | 28,46 | 31,37 |
| 64 | 28,29 | 30,99 |

As can be seen from the table, signals originating from the high copy number mouse GAPDH gene as well as the RPLI13A gene, which is present in 12 copies in the mouse genome, can be detected even if only 1 cell is used as a starting material.

Moreover, it can be observed that the cp values inversely correlate with the number of cells/per well. Thus, it can be obviously concluded that addition of the PCR reaction buffer and subsequent incubation for 10' at 95°C was obviously enough to lyse the cells in a quantitative manner.

### Example 2

### qPCR for amplification of the Kcnj2 gene from sorted mouse hybridoma cells

The experiment was essentially carried out as disclosed for example 1 with the alteration that primers and probe were designed to amplify the single copy mouse gene Kcnj2. Primers and probe were as follows:
Forward primer ctgtcttgccttcgtgctct (SEQ ID NO: 5)
Reverse primer agcagggctatcaaccaaaa (SEQ ID NO: 6)
UPL Probe (RocheApplied Science Cat. No: 04 688 996 001, No.76)

The table discloses the average cp values obtained for the different cell numbers analyzed:

| Number of cells | Average cp value |
|---|---|
| 1 | 38,31 |
| 2 | 36,64 |
| 4 | 36,26 |
| 8 | 36,10 |
| 16 | 33,93 |
| 32 | 32,88 |
| 64 | 34,03 |

As can be seen from the table, amplification signals originating from the single copy number mouse gene Kcnj2 can be obtained, even if only one cell is used as a starting material. In other words, the present invention provides a solution for amplification of single copy genes from single cell samples.

Moreover, it can be observed that the cp value increases, if the sample originates from a higher cell number such as 64 cells. This can be explained by the fact that due to the lysis within the PCR reaction buffer at 95°C, the concentration of cell debris within the given reaction volume increases and thus may inhibit amplification efficiency of the PCR reaction. It can be concluded that the present inventions is especially applicable for PCR on samples originating from lower cell numbers.

Furthermore, the following table discloses the cp values obtained from individual single cell samples:

| | | | | | | | |
|---|---|---|---|---|---|---|---|
| 40,00 | 36,96 | 36,71 | 35,92 | - | 40,00 | - | - |
| 37,71 | 40,00 | - | 40,00 | 39,56 | 37,19 | 37,22 | 38,50 |

As it can be deduced from the table, no amplification signals were obtained in about 4 out of 16 parallel reactions. Taking the results of example 2 into account, which proves that not every single sorting event results in the actual separation and delivery of a single cell into a reaction vessel this result is explainable. In other words, the fact that in some cases no amplificationsignal is observed is due to the fact that the individual wells did not contain a cell, but it is not due to the fact that the lysis and amplification procedure itself has a certain failure rate.

### Example 3

### qPCR for amplification of the Kcnj2 gene from sorted mouse hybridoma cells using microtiter plates containing dried reagents

The experiment was essentially performed as disclosed for example 2 with the following alterations:
10 µl of a solution containing the required primers and probe was filled into each well of a microtiter plate. The microtiter plate was incubated for 12h at 25°C and 200 mBar, and subsequently for 4 h at 25°C and 50 mBar, so that the primers and probes were dried onto the surface of each reaction well of the microtiter plate.

Subsequently, cell deposition was performed as follows:
1 cell equivalent/well of column 1-6
2 cell equivalents/well in column 7
4 cell equivalents/well in column 8
8 cell equivalents/well in column 9
16 cell equivalents/well in column 10
32 cell equivalents/well in column 11
64 cell equivalents/well in column 12

After addition of 20µl master mix, the real time PCR analysis was performed. The table discloses the average cp values obtained for the different cell numbers analyzed:

| Number of cells | Average cp value |
|---|---|
| 1 | 38,14 |
| 2 | 37,45 |
| 4 | 36,22 |
| 8 | 35,14 |
| 16 | 34,89 |
| 32 | 33,76 |
| 64 | 33,27 |

It is also important to note that from the 48 wells used for single cell analysis, only 10 amplification reactions were negative. These reactions were not included into the calculation of the average cp value.

### Example 4

### qPCR for amplification of the Kcnj2 gene from single mouse hybridoma cells using microtiter plates containing dried reagents

In order to analyze, how much percentage of cell equivalent actually corresponds to a living cell rather then to cellular debris, 3 x 30 sorted equivalents were deposited each on a microscopic slide and counted. 28, 28 and 29 cells, respectively could be identified by visual inspection through a microscope. This corresponds to 94% living cells versus 6 % cellular debris per cell equivalent (sorting event).

In the following the experiment was essentially performed on two microtiter plates as disclosed for example 3 with the alteration, that on both microtiter plates, each reaction well only contained a single cell equivalent. Results were as follows:

| Plate No. | Number of wells without detectable amplification | Percentage of wells with detectable amplification | Average cp value of wells with detectable amplification | Standard deviation cp value |
|---|---|---|---|---|
| 1 | 16/96 | 84% | 38,85 | 1,14 |
| 2 | 5/96 | 95% | 38,31 | 1,01 |

The results show that it is possible to perform single cell analysis according to the PCR method as provided by the present invention. Moreover, also if single cell analysis is intended, the primers and probe are dried onto the surface of the microtiter plate.

### Example 5

### 1-step-RT-PCR for detection of the ActB, and B2M and 1expression

Cells were sorted and disposed on a microtiter plate as disclosed in example 1, resulting in sample volume of less than 2 µl.

To each well, a 1x LC480 RNA Master Hydrolysis probes (Roche Applied Science Cat. No 04 991 885 001, containing T.th polymerase and a hot start aptamer) was added.

The master mix in addition contained the following primers and probes:
Row 1-2: 1-step RT-PCR of ActB
   0.4 µM Forward primer AAGGCCAACCGTGAAAAGAT (SEQ ID NO: 7)
   0.4 µM Reverse primer GTGGTACGACCAGAGGCATAC (SEQ ID NO: 8)
   0.2 µM UPL Probe (RocheApplied Science Cat. No: 56 )
Row 3-4: 1-step RT-PCR of B2M
   0.4 µM Forward primer TACGCCTGCAGAGTTAAGCA (SEQ ID NO: 9)
   0.4 µM Reverse primer GGTTCAAATGAATCTTCAGAGCA (SEQ ID NO: 10)
   0.2 µM UPL Probe (RocheApplied Science Cat. No: 117)
Row 5-6: 1-step RT-PCR of 18s RNA
   0.4 µM Forward primer GCCGCTAGAGGTGAAATTCTT (SEQ ID NO: 11)
   0.4 µM Reverse primer CGTCTTCGAACCTCCGACT (SEQ ID NO: 12)
   0.2 µM UPL Probe (Roche Applied Science Cat. No: 93)

On a first plate, 1-step-RT-PCR was performed in an LC480 real time PCR instrument according to the following thermocycling protocol:

| | | | |
|---|---|---|---|
| Preincubation: | 1x | 95°C | 30" |
| Denaturation | 45x | 95°C | 10" |

| | | | |
|---|---|---|---|
| Annealing | 45x | 60°C | 30" |
| Elongation | 45x | 72°C | 1" |
| Cooling ramp rates | 2.2°C/s | | |
| Heating ramp rates | 4.4°C/s | | |

On a second plate, 1-step-RT-PCR was performed in an LC480 real time PCR instrument according to a thermocycling protocol including a further preincubation step at 61°C.

| | | | |
|---|---|---|---|
| Preincubation: | 1x | 61°C | 3' |
| Preincubation: | 1x | 95°C | 30" |
| Denaturation | 45x | 95°C | 10" |
| Annealing | 45x | 60°C | 30" |
| Elongation | 45x | 72°C | 1" |
| Cooling ramp rates | 2.2°C/s | | |
| Heating ramp rates | 4.4°C/s | | |

Detection of amplification signals and calculation of cp values (low cp values indicating a high level of amplification) was performed according to the instructions of the Manufacturer's manuals.

In order to prove that the measured cp values actually reflect the detection of mRNA expression rather than DNA, the correct size of the amplicons was subsequently confirmed by means gel electrophoresis. The following table discloses the average cp values obtained for the different cell numbers analyzed.

| | **Without preincubation at 61°C** | | **With preincubation at 61°C** | |
|---|---|---|---|---|
| **Target/ Number of cells** | **MeanCp** | **STD Cp** | **MeanCp** | **STD Cp** |
| **Actb/1** | 32.06 | 0.60 | 30.55 | 0.35 |
| **Actb/2** | 31.60 | 1.12 | 29.12 | 0.50 |
| **Actb/4** | 31.15 | 0.71 | 28.71 | 0.47 |
| **Actb/8** | 29.92 | 0.11 | 27.78 | 0.07 |
| **Actb/16** | 29.13 | 0.30 | 27.05 | 0.39 |
| **Actb/32** | 28.24 | 0.22 | 25.98 | 0.23 |
| **Actb/64** | 26.47 | 0.13 | 24.11 | 0.01 |
| **B2M/1** | 36.04 | 0.38 | 31.64 | 0.00 |
| **B2M/2** | 36.13 | 0.43 | 30.99 | 0.06 |
| **B2M/4** | 35.54 | 0.24 | 30.88 | 0.22 |
| **B2M/8** | 34.64 | 0.48 | 30.69 | 0.25 |
| **B2M/16** | 34.52 | 0.01 | 30.21 | 0.13 |
| **B2M/32** | 34.19 | 0.13 | 30.15 | 0.08 |
| **B2M/64** | 34.59 | 0.07 | 30.13 | 0.10 |
| **18s/1** | 27.90 | 0.55 | 24.43 | 1.36 |
| **18s/2** | 27.27 | 0.32 | 23.59 | 0.74 |
| **18s/4** | 26.81 | 0.44 | 22.43 | 0.57 |
| **18s/8** | 25.74 | 0.19 | 22.49 | 0.21 |
| **18s/16** | 25.21 | 0.49 | 21.62 | 0.16 |
| **18s/32** | 24.66 | 0.18 | 20.78 | 0.25 |
| **18s/64** | 23.44 | 0.06 | 19.38 | 0.03 |

As can be seen from the table, expression of the genes tested can be detected in material originating from only one cell used as a starting material. It can be observed that the cp values inversely correlate with the number of cells/per well. The reverse transcription step takes place during the initial ramping, annealing and elongation steps of the thermocycling protocol.

As can be further deducted from the table, surprisingly the detection of expression is even more sensitive, in case of a preincubation step for 3 minutes at 61°C prior to the actual lysis at 95°C. Probably such a positive effect is due to the small sample volume of less than 2 µl, which results in an immediate drying and destruction of the few sorted cells, such that the cellular RNA becomes available for the reverse transcription reaction.

### Example 6

### Comparison between DNA PCR and 1-step-RT-PCR on the 18s target

Cells were sorted and disposed on a microtiter plate as disclosed in example 1 according to the following pipetting scheme:

For 3 identical plates No 1-3, 1x LC480 RNA Master Hydrolysis probes (Roche Applied Science Cat. No 04 991 885 001, containing T.th polymerase and a hot start aptamer) was added in order to amplify an RNA with a 1-step-RT PCR reaction. Primers were designed in such a way that during this reaction, both the RNA and its corresponding genomic DNA fragment were amplifiable. For a fourth plate No 4, a Real Time ready DNA probes master (Roche Applied Science Cat. No: 05 502 381 001) comprising a thermostable DNA dependent DNA Polymerase without any reverse transcriptase activity was used.

In addition the 4 set ups contained the following primers and probes suitable for amplification of 18s RNA and DNA
0.4 µM Forward primer GCCGCTAGAGGTGAAATTCTT (SEQ ID NO: 11)
0.4 µM Reverse primer CGTCTTCGAACCTCCGACT (SEQ ID NO: 12)
0.2 µM UPL Probe (Roche Applied Science Cat. No: 93)

For Plate 1, 1-step-RTPCR was performed with a preincubation step at 61°C in an LC480 real time PCR instrument according to the following thermocycling protocol:

| | | | |
|---|---|---|---|
| Predenaturation: | 1x | 95°C | 1' |
| Preincubation: | 1x | 61°C | 3' |
| Preincubation: | 1x | 95°C | 30" |
| Denaturation | 45x | 95°C | 10" |
| Annealing | 45x | 60°C | 30" |
| Elongation | 45x | 72°C | 1" |
| Cooling ramp rates | 2.2°C/s | | |
| Heating ramp rates | 4.4°C/s | | |

For plate 2 and 3, RT-PCR was performed without any preincubation at 61 °C:

| | | | |
|---|---|---|---|
| Preincubation: | 1x | 95°C | 30"(plate 2) or 2' (plate 3) |
| Denaturation | 45x | 95°C | 10" |
| Annealing | 45x | 60°C | 30" |
| Elongation | 45x | 72°C | 1" |
| Cooling ramp rates | 2.2°C/s | | |
| Heating ramp rates | 4.4°C/s | | |

The same protocol as for plate 3 was used for plate 4 in order to perform a PCR without any reverse transcription activity.

Detection of amplification signals and calculation of cp values (low cp values indicating a high level of amplification) was performed according to the instructions of the Manufacturer's manuals.

In order to prove that the measured cp values actually reflect the detection of mRNA expression rather than DNA, the correct size of the amplicons was subsequently confirmed by means gel electrophoresis. The following table discloses the average cp values obtained for the different cell numbers analyzed.

| | **Plate 1 RT-PCR** | | **Plate 2 RT-PCR without RT step With 30" preincubation** | | **Plate 3 RT-PCR without RT step With 2' preincubation** | | **Plate 4 PCR** | |
|---|---|---|---|---|---|---|---|---|
| **No of cells** | **MeanCp** | **STD Cp** | **MeanCp** | **STD Cp** | **MeanCp** | **STD Cp** | **MeanCp** | **STD Cp** |
| **1** | 25.24 | 1.11 | 27.90 | 0.55 | 29.13 | 0.70 | 34.18 | 0.68 |
| **2** | 23.97 | 0.54 | 27.27 | 0.32 | 28.48 | 0.47 | 32.99 | 0.41 |
| **4** | 23.74 | 0.86 | 26.81 | 0.44 | 27.67 | 0.17 | 31.98 | 0.38 |
| **8** | 22.48 | 0.14 | 25.74 | 0.19 | 26.95 | 0.21 | 30.93 | 0.23 |
| **16** | 21.82 | 0.14 | 25.21 | 0.49 | 25.63 | 0.15 | 29.05 | 0.36 |
| **32** | 19.92 | 0.12 | 24.66 | 0.18 | 25.18 | 0.26 | 28.16 | 0.04 |
| **64** | 18.89 | 0.01 | 23.44 | 0.06 | 23.87 | 0.05 | 27.60 | 0.33 |

As can be seen from the table, the results from the RT-PCR provide lower cp values as compared to results from the corresponding PCR reactions. This is indicative for a higher starting concentration of target nucleic acid, which according to the conditions as chosen results from amplification of both the respective RNA and its corresponding gene sequence within the RT-PCR set up.

When the RT-PCR results with 3 minutes at 61°C and no preincubation at 61°C are compared, it is obvious that a preincubation is not necessary but results in an increased sensitivity of RNA detection. As disclosed in example 5 the latter observed effect may be due to the small sample volume of less than 2 µl, which results in an immediate drying and destruction of the few sorted cells, such that the cellular RNA becomes available for the reverse transcription reaction.

## Claims

1. Method for amplification of an RNA target nucleic acid, comprising the steps of
a) transfering a liquid sample with a first volume of less than 2 µl comprising one or more living eukaryotic cells into a vessel,
b) adding to said vessel a one-step RT-PCR reaction buffer comprising a thermostable DNA polymerase capable of performing a one-step RT-PCR, and dNTPs, with a second volume, whereas said second volume is at least 2x as large as said first volume,
b') incubating said sample at a temperature between 37°C and 65°C for 30 seconds to 5 min.
c) heating said vessel for at least 20 seconds at at least 90°C,
d) amplifying said target in said vessel by means of a polymerase chain reaction with a thermostable DNA polymerase capable of performing a one-step RT-PCR without performance of an intermediate purification step,
wherein the ratio of the number of said living cells of step a) versus the liquid volume in which the polymerase chain reaction of step d) is not greater than 2 cells/µl, said ratio is at least 1 cell/25/µl.

2. Method according to claim 1, **characterized in that** said RT-PCR reaction buffer of step b) in addition comprises at least one pair of amplification primers and optionally either at least one labeled hybridization probe or a double strand DNA binding fluorescent compound.

3. Method according claim 1, **characterized in that** said vessel comprises a dry composition of at least one pair of PCR amplification primers and optionally either at least one labeled hybridization probe or a double strand DNA binding fluorescent compound.

4. Method according to claims 1-2, **characterized in that** the activity of said thermostable DNA polymerase is thermally activated during step c).

5. Method according to claims 1-4, wherein said polymerase is Tth Poylmerase.

6. Method according to claims 1-5, **characterized in that** prior to step a) said liquid comprising at least one or more living cells has been gained by a cell sorting method.

## Patentansprüche

1. Verfahren zur Amplifikation einer RNA-Zielnukleinsäure, umfassend die Schritte
a) Überführen einer Flüssigkeitsprobe mit einem ersten Volumen von weniger als 2 µl, die eine oder mehrere lebende eukaryontische Zellen umfasst, in ein Gefäß,
b) Zugeben eines Ein-Schritt-RT-PCR-Reaktionspuffers mit einem zweiten Volumen, der eine thermostabile DNA-Polymerase, die zur Ausführung einer Ein-Schritt-RT-PCR in der Lage ist, und dNTPs umfasst, wobei das zweite Volumen wenigstens 2x so groß wie das erste Volumen ist, in das Gefäß,
b') Inkubieren der Probe bei einer Temperatur zwischen 37°C und 65°C über einen Zeitraum von 30 Sekunden bis 5 min,
c) Erhitzen des Gefäßes über einen Zeitraum von wenigstens 20 Sekunden bei wenigstens 90°C,
d) Amplifizieren des Ziels in dem Gefäß mittels einer Polymerasekettenreaktion mit einer thermostabilen DNA-Polymerase, die zur Ausführung einer Ein-Schritt-RT-PCR in der Lage ist, ohne Ausführung eines dazwischen geschalteten Reinigungsschritts,
wobei das Verhältnis der Anzahl der lebenden Zellen in Schritt a) gegenüber dem Flüssigkeitsvolumen, in dem die Polymerasekettenreaktion in Schritt d) nicht höher als 2 Zellen/µl liegt, wobei das Verhältnis wenigstens 1 Zelle/25 µl beträgt.

2. Verfahren nach Anspruch 1, **dadurch gekennzeichnet, dass** der RT-PCR-Reaktionspuffer in Schritt b) zusätzlich wenigstens ein Paar Amplifikationsprimer und gegebenenfalls entweder wenigstens eine markierte Hybridisierungssonde oder eine Doppelstrang-DNA bindende Fluoreszenzverbindung umfasst.

3. Verfahren nach Anspruch 1, **dadurch gekennzeichnet, dass** das Gefäß eine trockene Zusammensetzung von wenigstens einem Paar PCR-Amplifikationsprimer und gegebenenfalls entweder wenigstens einer markierten Hybridisierungssonde oder einer Doppelstrang-DNA bindenden Fluoreszenzverbindung umfasst.

4. Verfahren nach Anspruch 1-2, **dadurch gekennzeichnet, dass** die Aktivität der thermostabilen DNA-Polymerase während Schritt c) thermisch aktiviert wird.

5. Verfahren nach Anspruch 1-4, wobei es sich bei der Polymerase um Tth-Polymerase handelt.

6. Verfahren nach Anspruch 1-5, **dadurch gekennzeichnet, dass** vor Schritt a) die wenigstens eine oder mehrere lebende Zellen umfassende Flüssigkeit mit einem Zellsortierungsverfahren erhalten wurde.

## Revendications

1. Procédé d'amplification d'un ARN à titre d'acide nucléique cible, comprenant les étapes dans lesquelles :
a) on transfère un échantillon liquide avec un premier volume inférieur à 2 µl comprenant une ou plusieurs cellules eucaryotes vivantes, dans un récipient ;
b) on ajoute audit récipient, un tampon de réaction PCR en temps réel à une seule étape comprenant une ADN polymérase thermostable capable de mettre en oeuvre une PCR en temps réel à une seule étape et des dNTP, avec un deuxième volume, ledit deuxième volume étant néanmoins au moins deux fois plus grand que ledit premier volume ;
b') on incube ledit échantillon à une température entre 37 °C et 65 °C pendant un laps de temps de 30 secondes à 5 minutes ;
c) on chauffe ledit récipient pendant au moins 20 secondes à au moins 90 °C ;
d) on amplifie ladite cible dans ledit récipient au moyen d'une réaction en chaîne par polymérase avec une ADN polymérase thermostable capable de mettre en oeuvre une PCR en temps réel à une seule étape sans passer par une étape de purification intermédiaire ;
dans lequel le rapport entre le nombre desdites cellules vivantes de l'étape a) et le volume de liquide dans lequel a lieu la réaction en chaîne par polymérase de l'étape d) n'est pas supérieur à 2 cellules/µl, ledit rapport s'élevant à au moins 1 cellule/25 µl.

2. Procédé selon la revendication 1, **caractérisé en ce que** ledit tampon de réaction PCR en temps réel de l'étape b) comprend en outre au moins une paire d'amorces d'amplification et de manière facultative, soit au moins une sonde d'hybridation marquée, soit un composé fluorescent se liant à de l'ADN double brin.

3. Procédé selon la revendication 1, **caractérisé en ce que** ledit récipient comprend une composition sèche d'au moins une paire d'amorces d'amplification par PCR et de manière facultative, soit au moins une sonde d'hybridation marquée, soit un composé fluorescent se liant à de l'ADN double brin.

4. Procédé selon les revendications 1 à 2, **caractérisé en ce que** l'activité de ladite ADN polymérase thermostable est activée par voie thermique au cours de l'étape c).

5. Procédé selon les revendications 1 à 4, **caractérisé en ce que** ladite polymérase est la polymérase Tth.

6. Procédé selon les revendications 1 à 5, **caractérisé en ce que**, avant l'étape a), on obtient ledit liquide comprenant au moins une ou plusieurs cellules vivantes à partir d'un procédé de triage cellulaire.
